# EUROPÄISCHE PATENTSCHRIFT

(11) **EP 2 244 703 B1**
(45) Veröffentlichungstag und Bekanntmachung des Hinweises auf die Patenterteilung: **28.09.2011**
(21) Anmeldenummer: 08868390.9
(22) Anmeldetag: 12.12.2008
(51) Int. Cl.: A61K 31/192, A61P 25/00, A61P 29/02

(54) **ARZNEIMITTEL SOWIE DEREN HERSTELLUNG UND VERWENDUNG BEI DER BEHANDLUNG VON SCHMERZHAFTEN NEUROPATHIEN**
PHARMACEUTICALS AND THE PRODUCTION AND USE THEREOF IN THE TREATMENT OF PAINFUL NEUROPATHIES
MÉDICAMENT, SA FABRICATION ET SON UTILISATION POUR LE TRAITEMENT DE NEUROPATHIES DOULOUREUSES

(30) Priorität: 21.12.2007 DE 102007062139
(43) Veröffentlichungstag der Anmeldung: 03.11.2010
(73) Patentinhaber: Horizon Pharma AG, 4153 Reinach (CH)
(72) Erfinder: GEISSLINGER, Gerd, 65812 Bad Soden (DE); TEGEDER, Irmgard, 60598 Frankfurt (DE)
(74) Vertreter: Wagner, Jutta
(86) Internationale Anmeldenummer: PCT/EP2008/010592
(87) Internationale Veröffentlichungsnummer: WO 2009/083115

(56) Entgegenhaltungen:
- WO-A-98/20864
- WO-A-2005/102338
- WO-A-2008/036733
- LOETSCH, JOERN ET AL: "Effects of flurbiprofen enantiomers on pain -related chemo-somatosensory evoked potentials in human subjects" BRITISH JOURNAL OF CLINICAL PHARMACOLOGY , 40(4), 339-46 CODEN: BCPHBM; ISSN: 0306-5251, 1995, XP008104450

## Beschreibung

Die vorliegende Erfindung bezieht sich auf Tarenflurbil (vorgeschlagene INN Bezeichnung; frühere Bezeichnung R-Flurbiprofen) in reiner oder gegenüber dem Racemat in angereicherter Form als Wirkstoff enthaltende Arzneimittel, auf die Herstellung und Verwendung der Arzneimittel in systemisch oder topisch applizierbarer pharmazeutischer Zubereitung als schnell oder modifiziert freisetzende Formulierungen zur Behandlung schmerzhafter Neuropathien oder neuropathischer Schmerzen bei Säugern, insbesondere beim Menschen.

### Hintergrund der Erfindung

Neuropathien sind krankhafte Nervenzustände, die zwei sehr verschiedene Erkrankungsarten betreffen. Eine Erkrankungsart ist die Erkrankung aufgrund einer Nervenschädigung im Sinne einer somatischen Krankheit. Die andere Erkrankungsart betrifft das Nervensystem im Sinne einer reizbaren Schwäche (z.B. Nervosität, Neurasthenie, Neurose). Die letztgenannte Bedeutung ist mehr historisch zu sehen, heute benutzt man dafür eher den Ausdruck psychische Erkrankung.

Somatisch bedingte Neuropathien können verschiedene Ursachen haben, z.B. Nervendurchtrennung bei Amputation oder Unfall, Durchblutungsstörungen bei arterieller Verschlusskrankheit oder Diabetes mellitus, mechanisch schädigende Einwirkungen (Traumen) auf den Nerv, Tumore, metabolische (stoffwechselbedingte) Störung oder Schädigung von Nerven durch Diabetes mellitus, Vitaminmangel, Leber- und Nierenerkrankungen, infektiöse Erkrankungen wie Herpes zoster, toxische Erkrankungen durch Alkohol, Schwermetalle, Medikamente, zyklische Kohlenwasserstoffe, autoimmunologische Erkrankungen, vor allem beim Guillain-Barré-Syndrom, und zentrale, also das Rückenmark oder das Gehirn betreffende, Störungen oder Schädigungen.

Abhängig von der Krankheitsursache stehen zur Behandlung der Neuropathie zahlreiche Möglichkeiten zur Verfügung. Gegenwärtig angewendete Therapiealgorithmen berücksichtigen die Therapie der zugrunde liegenden Ursache, die pharmakologische und nicht pharmakologische Therapie der Krankheitssymptome sowie die supportive psychologische Therapie.

Da bei somatischen Neuropathien das schmerzleitende System selbst gestört oder geschädigt ist, treten häufig Nerveneigenschmerzen, die so genannten neuropathischen Schmerzen, auf. Dieser Schmerztyp ist charakterisiert durch brennende, blitzartige Spontanschmerzen, einschießende Schmerzattacken und evozierte Schmerzen (Allodynie, Hyperalgesie), aber auch durch Parästhesien und Hypästhesien.

Neuropathische Schmerzen unterscheiden sich wegen der unterschiedlichen Ursachen und der unterschiedlichen Entwicklung des Krankheitsgeschehens gänzlich von den Nozizeptorschmerzen. Nozizeptorschmerzen treten im Gegensatz zu neuropathischen Schmerzen nach Gewebeschädigungen oder Entzündungen auf, bei denen die peripheren und zentralen Nervenstrukturen intakt sind. Somit sind bei Nozizeptorschmerzen die Auslösung, Weiterleitung und zentrale Verarbeitung der Schmerzimpulse durch das periphere und zentrale Nervensystem, die sogenannte Nozizeption, voll funktionsfähig. Zu den Nozizeptorschmerzen gehören z.B. alle chronischen Entzündungsschmerzen, viszerale Schmerzen, viele Komponenten chronischer Rückenschmerzen und die meisten Komponenten von Tumorschmerzen.

Wegen der Unterschiede in der Krankheitsursache, im Krankheitsverlauf und in der medikamentösen Therapie hat das Committee for Proprietary Medicinal Products (CPMP) der European Agency for the Evaluation of Medicinal Products (EMEA) unterschiedliche amtliche Richtlinien zur klinischen Entwicklung von Arzneimitteln zur Behandlung neuropathisch bedingter Schmerzen [Guideline on Clinical Medicinal Products intended for the Treatment of Neuropathic Pain, CPMP/EWP/252/03 Rev.1, 24 January 2007] und nozizeptiver Schmerzen [Note for Guidance on Clinical Investigation of Medicinal Products for Treatment of Nociceptive Pain, CPMP/EWP/612/00, 21 November 2002] erlassen.

Schmerzhafte Neuropathien beeinträchtigen erheblich die Lebensqualität des Patienten und stellen ein bedeutendes gesundheitsökonomisches Problem dar. Die Punktprävalenz der schmerzhaften Neuropathien in der Allgemeinbevölkerung wird nach einer Erhebung in sechs europäischen Ländern auf ca. 5% geschätzt [McDermott AM, Tölle TR, Rowbotham DJ, Schaefer CP, Dukes EM: The burden of neuropathic pain: results from a cross-sectional survey. Eur J Pain 2006; 10(2): 127-135.]. Bei dem "Second International Congress on Neuropathic Pain" der International Association for the Study of Pain (IASP) vom 7. bis 10. Juni 2007 in Berlin wurde festgestellt, dass in Deutschland bei 8% der Patienten mit Hirninfarkten, 20% der Diabetiker, 28% der Patienten mit multipler Sklerose, ca. 33% der Patienten mit Tumorschmerzen, 37% der Patienten mit Rückenschmerzen und 67% der Patienten mit Rückenmarksverletzungen neuropathische Schmerzen auftreten.

Schmerzen in Folge einer Neuropathie bedürfen einer anderen medikamentösen Therapie als Nozizeptorschmerzen. Deshalb ist es essentiell, vor Behandlungsbeginn das Vorliegen neuropathischer Schmerzen zu diagnostizieren und diese von den in der medizinischen Praxis häufiger vorkommenden Nozizeptorschmerzen zu unterscheiden. Zur sicheren Diagnose neuropathischer Schmerzen stehen mehrere validierte Verfahren zur Verfügung [Übersicht in: Baron, R: Diagnostik und Therapie neuropatischer Schmerzen (Detection of neuropathic pain syndroms), Deutsches Ärzteblatt 103, Heft 41, 2006, 2720-30]. Die Diagnosemöglichkeiten reichen von klinisch orientierten Diagnosen [Dworkin RH, Backonja M, Rowbotham MC, Allen RR, Argoff CR, Bennett GJ et al.: Advances in neuropathic pain: diagnosis, mechanisms, and treatment recommendations. Arch Neurol 2003; 60(11): 1524-34. Cruccu G, Anand P, Attal N, Garcia-Larrea L, Haanpaa M, Jorum E et al.: EFNS guidelines on neuropathic pain assessment. Eur J Neurol 2004; 11 (3): 153-62. Jensen TS, Baron R: Translation of symptoms and signs into mechanisms in neuropathic pain. Pain 2003; 102(1-2):1-8] bis zu einfachen Fragebögen [Freynhagen R, Baron R, Gockel U, Tölle TR: painDETECT - a new screening questionaire to identify neuropathic components in patients with back pain. Curr med Res Opin 2006; 22(10): 1911-20].

Zur Pharmakotherapie neuropathischer Schmerzen stehen nach aktuellen Therapierichtlinien [Baron R, Ludwig J, Binder A: Therapie Tabellen Neurologie/Psychiatrie Nr. 29, Mai 2006, Neuropathische Schmerzen, Westermayer Verlag, Pentenried] folgende Arzneimittel zur Verfügung: Antidepressiva (z.B. Amitriptylin, Nortriptylin; Desipramin, Maprotilin, Venlafaxin, Duloxetin, Bupropion), Antikonvulsiva (z.B. Carbamazepin, Oxcarbazepin, Lamotrigin, Gabapentin, Pregabalin), Opioide (z.B. Tramadol, Morphin, Oxycodon), Cannabinoide (z.B. Tetrahydro-Cannabinol), Myotonolytika (z.B. Baclofen), und NMDA- (= N-Methyl-D-Aspartat-) Antagonisten (z.B. Dextromethorphan, Ketamin, Memantine), Radikalfänger (z.B. Alpha-Liponsäure). Für topisch anzuwendende Arzneimittel kommen lokale Analgetika und Lokalanästhetika (z.B. Capsaicin, Lidocain und Benzocain) in Frage.

Zur Pharmakotherapie nozizeptiver Schmerzen werden Opioid-Analgetika und nicht-opioide Analgetika eingesetzt. Zu den nicht-opioiden Analgetika zählen unter anderem die analgetisch, antiphlogistisch und antipyretisch wirksamen nichtsteroidalen Antiphlogistika (NSAIDs) inklusive der COX-2 selektiven Inhibitoren. NSAIDs sind nicht in die aktuellen Therapieempfehlungen und in die amtlichen Richtlinien der EMEA zur Behandlung schmerzhafter Neuropathien aufgenommen worden, weil sie sich als unwirksam erwiesen haben und deshalb nur Nebenwirkungen verursachen würden. Dies gilt z. B. für Ibuprofen [Baron, R: Diagnostik und Therapie neuropatischer Schmerzen (Detection of neuropathic pain syndroms), Deutsches Ärzteblatt 103, Heft 41, 2006, 2720-30. Max, MB et al.: Association of pain relief with drug side effects in postherpetic neuralgia: a single-dose Study of clonidine, codeine, ibuprofen, and placebo, Clin Pharmacol Ther. 1988;43(4), 363-71] sowie Diclofenac, Indomethacin und Aspirin [Hempenstall, K et al.: Analgesic therapy in in postherpetic neuralgie: A quantitative systematic review. PLoS Med. 2005; 2(7),1-27].

Von den derzeit zur Verfügung stehenden Schmerzmitteln eignen sich somit nach den aktuellen Therapieempfehlungen nur Opioid-Analgetika sowohl zur Behandlung von schmerzhaften Neuropathien als auch zur Behandlung nozizeptiver Schmerzen. Alle anderen Wirkstoffe sind wegen der Unterschiede in der Schmerzursache und aufgrund ihres Wirkungsmechanismus entweder nur zur Behandlung schmerzhafter Neuropathien oder nur zur Behandlung nozizeptiver Schmerzen geeignet. Dementsprechend müssen gemischte Schmerzsyndrome, bestehend aus neuropathischen und aus nozizeptiven Schmerzen, regelmäßig mit den jeweils für den betreffenden Schmerztyp geeigneten Wirkstoffen individuell behandelt werden.

Einige der bekannten NSAIDs enthalten ein asymmetrisches Kohlenstoffatom und kommen deshalb als R-Enantiomer und als S-Enantiomer vor. Dazu gehört auch die Wirkstoffklasse der 2-Arylpropionsäuren, der z.B. bekannte Substanzen wie Ibuprofen, Flurbiprofen, Ketoprofen, Naproxen und Tiaprofensäure angehören. Ibuprofen und Ketoprofen werden sowohl als Racemat (50% S-Enantiomer, 50% R-Enantiomer), als auch als reines S-Enantiomer therapeutisch eingesetzt, wobei nur das S-Enantiomer als wirksam gilt. Naproxen findet ausschließlich als S-Enantiomer in Arzneimitteln Verwendung. Flurbiprofen und Tiaprofensäure werden bisher therapeutisch nur als Racemat eingesetzt.

Nach dem Stand der Technik ist von einigen 2-Arylpropionsäuren inzwischen bekannt, dass sie entgegen der früheren wissenschaftlichen Erkenntnis nicht nur als S-Enantiomer pharmakologisch wirksam sind, sondern auch als R-Enantiomer erwünschte pharmakologische Effekte, insbesondere einen analgetischen Effekt, zeigen, siehe z.B. DE 40 28 906, EP 0 607 128, US 5,200,198 und US 5,206,029. Diese Dokumente befassen sich nur mit nozizeptiven Schmerzen.

In WO 00/13684 wird beschrieben, dass einige R-(2)-Arylpropionsäuren, bevorzugt Tarenflurbil, nach Applikation von Dosen, die höher als die analgetisch wirksamen Dosen liegen, entzündungshemmend wirken, wobei als Wirkungsweise eine Hemmung der Induktion von COX-2 auf der Ebene der mRNA angegeben wird.

In EP 1 154 766 wird die Verwendung von R-(2)-Arylpropionsäuren, bevorzugt von Tarenflurbil, zur Herstellung von Arzneimitteln zur Bekämpfung von rheumatischen Erkrankungen, Asthma, Schock, entzündlichen Darmerkrankungen, Strahlenschäden, Arteriosklerose und Abstoßungsreaktionen nach Gewebe- oder Organtransplantationen beansprucht, wobei die Wirkung bei diesen Erkrankungen auf der Hemmung der Aktivierung des Transkriptionsfaktors NFkappaB beruhen soll.

Weitere Anwendungen von R-(2)-Arylpropionsäuren, bevorzugt von Tarenflurbil, als chemopräventive Wirkstoffe zur Behandlung neoplastischer Krankheiten (colorektaler Krebs) sowie zur Behandlung von cystischer Fibrose und Alzheimer Krankheit sind in WO 98/09603 beschrieben.

EP 1 322 305 beansprucht die Verwendung von R-(2)-Arylpropionsäuren, bevorzugt von Tarenflurbil, zur Behandlung von chronisch-destruktiven Knorpel- und Gelenkserkrankungen bei Rheuma.

Für Ketoprofen ist beschrieben, dass nach spinaler Applikation von R-Ketoprofen oder von Mischungen aus Morphin und S-Ketoprofen bei Ratten ein inhibitorischer Effekt auf die taktile Allodynie ausgelöst wird. Daraus wird geschlossen, dass die spinale Applikation dieser Substanzen zur Behandlung neuropathischer Schmerzen geeignet sein könnte [Ossipov MH, Jerussi TP, Ren K, Sun H, Porreca F: Differential effects of spinal (R)-ketoprofen and (S)-ketoprofen against signs of neuropahtic pain and tonic nociception: evidence for a novel mechanism of action of (R)-ketoprofen against tactile allodynia. Pain. 2000 Aug;87(2):193-9]. Das verwendete Schmerzmodell legt nahe, dass nicht rein neuropathische Schmerzen erfasst wurden, sondern eher nozizeptive Schmerzen oder gemischte Schmerzen, bestehend aus nozizeptiven und neuropathischen Schmerzen. Die im Tierexperiment gewählte spinale Applikation ist wegen des hohen Aufwandes nur begrenzt klinisch anwendbar. Diese Applikationsart würde nur die Behandlung von zentralen neuropathischen Schmerzen zulassen. Eine Behandlung von peripheren neuropathischen Schmerzen wäre nicht möglich. Die Daueranwendung dieser Applikationsart ist nur mit einem hohen technischen Aufwand, z.B. mit implantierbaren Pumpen möglich. Eine pharmakologische Wirkung dieser Substanz nach anderen, in der therapeutischen Praxis für die Langzeittherapie üblichen Applikationsarten, ist nicht beschrieben. Die Ergebnisse dieser wissenschaftlichen Veröffentlichung sind die Basis für US 6,620,851 B2, in der Verfahren zur Behandlung neuropathischer Schmerzen und anderer Störungen unter Verwendung von R(-)-Ketoprofen beansprucht werden. Orale Darreichungsformen werden genannt, ohne dass deren Wirksamkeit belegt wird. Die Wirksamkeit von reinem, oral verabreichtem R-Ketoprofen ließe sich jedoch weder an der Ratte, noch beim Menschen belegen, weil bei beiden Spezies R-Ketoprofen nach oraler Applikation in einem erheblichen Ausmaß zu S-Ketoprofen invertiert wird. Dadurch ist eine möglicherweise vorhandene Wirkung von R-Ketoprofen von der stärkeren Wirkung von S-Ketoprofen nicht zu trennen. Die Bioinversion von R-Ketoprofen zu S-Ketoprofen würde bei oraler Gabe außerdem zu den bekannten unerwünschten Wirkungen von S-Ketoprofen führen und somit die möglicherweise bessere Verträglichkeit von R-Ketoprofen zunichte machen.

Eine Übertragung der für R-Ketoprofen gefundenen Wirkung auf andere R-Arylpropionsäuren ist für den Fachmann aufgrund der bei bisherigen Untersuchungen gefundenen sehr verschiedenen Wirkungen bzw. Wirkungsstärken und vor allem der von Spezies zu Spezies und von Arylpropionsäure zu Arylpropionsäure stark unterschiedlichen Inversion nicht möglich. Insoweit sind die R-Arylpropionsäuren im Unterschied zu den S-Enantiomeren nicht als einheitliche Wirkstoffklasse anzusehen.

Es besteht weiterhin Bedarf an Therapieverfahren bzw. Arzneimitteln, die sich für die Behandlung schmerzhafter Neuropathien eignen.

### Zusammenfassung der Erfindung

Überraschend wurde nun gefunden, dass die Anwendung von Tarenflurbil in pharmakologischen Modellen zum neuropathischen Schmerz bei der Ratte zu einer signifikanten Reduzierung der evozierten neuropathischen Schmerzen führt.

Das bei nozizeptiven Schmerzen sehr wirksame S-Flurbiprofen zeigte diese Wirkung erwartungsgemäß nicht. Ratten sind nach bisheriger Erkenntnis die einzige etablierte Labortierspezies, die Tarenflurbil *in vivo* kaum zu S-Flurbiprofen invertiert. Deshalb eignet sich diese Spezies dazu, selektiv die Wirkungen von Tarenflurbil zu bestimmen, wobei die gefundenen Effekte auf den Menschen übertragbar sind.

Daher betrifft die vorliegende Erfindung ein Mittel zur Anwendung in der Behandlung von schmerzhafter Neuropathie; von schmerzhafter Neuropathie, die gleichzeitig von nozizeptiven Schmerzzuständen begleitet wird; von peripherem bzw. vorwiegend peripherem neuropathischem Schmerz; oder von zentralem bzw. vorwiegend zentralem, neuropathischem Schmerz bei einem Säuger, insbesondere beim Menschen.

Insbesondere betrifft die Erfindung ein Mittel zur Anwendung in der Behandlung von folgenden neuropathischen Schmerzen bzw. von neuropathischem Schmerz, der eine aus der Gruppe folgender Ursachen ausgewählte Ursache hat: Systemische Erkrankungen, z.B. diabetische Neuropathie; Arzneimittel-induzierte Läsionen, z.B. Neuropathie aufgrund von Chemotherapie; Traumatisches Syndrom und Entrapment-Syndrom; Läsionen der Nervenwurzeln und der Posteriorganglien; Neuropathien nach HIV Infektionen; Neuralgien nach Herpesinfektionen; Nervenwurzelavulsionen; Läsionen der Cranialnerven; Craniale Neuralgien, z.B., Trigeminalneuralgie; neuropathischer Krebsschmerz; Phantomschmerz; Kompression der peripheren Nerven, Nerven-Plexus und Nervenwurzeln; paraneoplastische periphere Neuropathie und Ganglionopathie; Komplikationen von Krebstherapien, z.B. Chemotherapie, Bestrahlung und chirurgische Eingriffe; komplexes regionales Schmerzsyndrom; Läsionen des Typs I (vormals bekannt als sympathische Reflexdystrophie); und Läsionen des Typs II (in etwa der Causalgie entsprechend).

Weiter betrifft die Erfindung ein Mittel zur Anwendung in der Behandlung von neuropathischem Schmerz mit einer Ursache, die ausgewählt ist aus der folgenden Gruppe von Ursachen: Zerebrale Läsionen, die vorwiegend thalamisch sind; Infarkt, z.B. thalamischer Infarkt oder Hirnstamminfarkt; zerebrale Tumore oder Abszesse, die den Thalamus oder den Hirnstamm komprimieren; Multiple Sklerose; Hirnoperationen, z.B. Thalamotomie bei motorischen Störungen; Läsionen des Rückenmarks; Verletzungen des Rückenmarks; Operationen am Rückenmark, z.B. anterolaterale Cordotomie; ischämische Läsionen; Anteriore Spinalarterie-Syndrom; Wallenbergs Syndrom; und Syringomyelie.

Dem gemäß wird enantiomerenreines oder im wesentlichen enantiomerenreines Tarenflurbil bzw. ein pharmazeutisch verträgliches Salz oder Derivat davon, oder Tarenflurbil bzw. pharmazeutisch verträgliches Tarenflurbilderivat oder -salz, das gegenüber Flurbiprofen-Racemat bzw. einem Racemat des genannten Salzes oder Derivates angereichert ist, an einen Säuger, insbesondere an einen Menschen, verabreicht, der unter den genannten schmerzhaften Neuropathien oder neuropathischen Schmerzen leidet.

Weiterhin betrifft die vorliegende Erfindung die Verwendung von Tarenflurbil bzw. einem pharmazeutisch verträglichen Salz oder Derivat davon in enantiomerenreiner bzw. im wesentlichen enantiomerenreiner oder gegenüber Flurbiprofen-Racemat bzw. einem Racemat des genannten Salzes oder Derivates angereicherter Form zur Herstellung eines Arzneimittels zur Behandlung der vorstehend genannten schmerzhaften Neuropathien oder neuropathischen Schmerzen bei einem Säuger, insbesondere bei einem Menschen.

Außerdem betrifft die Erfindung Tarenflurbil bzw. ein pharmazeutisch verträgliches Salz oder Derivat davon in enantiomerenreiner bzw. im wesentlichen enantiomerenreiner oder gegenüber Flurbiprofen-Racemat bzw. einem Racemat des genannten Salzes oder Derivates angereicherter Form, und Arzneimittel bzw. Arzneimittelzusammensetzungen, die diese enthalten, zur Verwendung für die Behandlung der vorstehend genannten schmerzhaften Neuropathien oder neuropathischen Schmerzen bei einem Säuger, insbesondere bei einem Menschen.

### Kurze Beschreibung der Abbildungen

Figur 1a, 1b und 1c zeigen die Dosis-abhängigen Wirkungen von Tarenflurbil (= R-Flurbiprofen; RF) im Spared Nerve Injury-Modell (SNI-Modell; linke Graphen) und im Chronic Constriction Injury-Modell (CCI-Modell; rechte Graphen) von peripherem neuropathischem Schmerz, verglichen mit den Wirkungen von S-Flurbiprofen (SF), Gabapentin (Gaba) und Vehikel (Vehicle).

Figur 2 zeigt die Wirkungen einer Behandlung ab dem ersten postoperativen Tag mit Tarenflurbil (= R-Flurbiprofen; RF), verabreicht in einer Dosis von 9 mg/kg zweimal täglich i.p., im Vergleich zu Vehikel im Spared Nerve Injury-Modell von peripherem neuropathischen Schmerz.

Figur 3 zeigt die Wirkungen von Tarenflurbil (9 mg/kg Einzeldosis i.p.) im Vergleich zu Vehikel in unbehandelten Ratten.

### Detaillierte Beschreibung der Erfindung

Obwohl NSAIDs bei der Behandlung nozizeptiver Schmerzen und entzündlicher Erkrankungen eine breite Anwendung finden, haben sie sich in bisherigen Untersuchungen bei der Behandlung von schmerzhaften Neuropathien als unwirksam erwiesen und sind deshalb in den Therapiealgorithmen zur Behandlung von Schmerzen bei Neuropathien nicht enthalten.

Überraschenderweise wurde im Rahmen der vorliegenden Erfindung gefunden, dass Tarenflurbil, das R-Enantiomer des als Racemat im Handel befindlichen NSAID Flurbiprofen, bei der Behandlung schmerzhafter Neuropathien schmerzlindernd wirksam ist. Die Wirkung von Tarenflurbil bei schmerzhaften Neuropathien ist selektiv und wird durch das S-Enantiomer von Flurbiprofen nicht hervorgerufen.

Die im Rahmen der vorliegenden Erfindung durchgeführten pharmakologischen Untersuchungen zeigen die dosisabhängige Wirkung von Tarenflurbil bei pharmakologisch anerkannten Modellen zu schmerzhaften Neuropathien. Die Wirkstärke liegt bei ausreichender Dosierung im Bereich der Wirkstärke von 25 mg/kg Körpergewicht Gabapentin als Referenzsubstanz. S-Flurbiprofen zeigt bei diesen Modellen keine signifikante Wirkung.

Tarenflurbil (bzw. ein pharmazeutisch verträgliches Salz oder Ester davon) enthaltende Arzneimittel können im Rahmen der Erfindung somit bei allen Formen der eingangs beschriebenen schmerzhaften Neuropathien oder neuropathischen Schmerzen als alleinige Therapie oder in Kombination mit anderen Arzneimitteln oder Behandlungsverfahren eingesetzt werden. Die Dosierung sollte in Anlehnung an die aktuellen Therapierichtlinien für die bekannten Wirkstoffe erfolgen. Das bedeutet, dass die Dosierung individuell, abhängig von Wirksamkeit und Nebenwirkungen erfolgen soll.

Bei der Behandlung von schmerzhaften Neuropathien bzw. neuropathischem Schmerz wird unter Wirksamkeit eine Schmerzreduktion um mindestens 30 bis 50% verstanden. Diese sollte vorzugsweise erst nach einer Behandlungsdauer von zwei bis vier Wochen unter hohen Tagesdosen beurteilt werden. Danach kann die Dosis nach oben oder nach unten angepasst oder eine Kombinationstherapie mit den bekannten Wirkstoffen eingeleitet werden.

Da die unerwünschten Arzneimittelwirkungen von Flurbiprofen-Racemat im Wesentlichen durch das S-Enantiomer verursacht werden, ist es im Rahmen der Erfindung besonders vorteilhaft, Tarenflurbil bzw. dessen pharmazeutisch verträglichen Salze oder Ester, in enantiomerenreiner oder im wesentlichen enantiomerenreiner Form zu verwenden bzw. zu verabreichen. Ebenfalls geeignet sind Formen von Tarenflurbil bzw. von dessen pharmazeutisch verträglichen Salzen oder Ester bei denen das Tarenflurbil bzw. das pharmazeutisch verträgliche Salz oder Ester gegenüber der entsprechenden racemischen Form, bestehend aus 50% Tarenflurbil bzw. pharmazeutisch verträglichem Salz oder Ester und 50% S-Flurbiprofen bzw. pharmazeutisch verträglichem Salz oder Ester davon, angereichert ist. Je höher der enantiomere Überschuss an Tarenflurbil bzw. pharmazeutisch verträglichem Salz oder Ester gegenüber dem entsprechenden S-Enantiomer ist, desto höher können daraus hergestellte Arzneimittel dosiert werden, um z.B. die erwünschte analgetische Wirkung bei der Behandlung schmerzhafter Neuropathien, verbunden mit einer möglichst geringen Häufigkeit und Schwere unerwünschter Arzneimittelwirkungen zu erzielen.

Insbesondere geeignet sind Formen von Tarenflurbil bzw. Formen des pharmazeutisch verträglichen Salzes oder Esters davon, bei denen das Tarenflurbil bzw. sein pharmazeutisch verträgliches Salz oder Ester in einem Molverhältnis zu S-Flurbiprofen bzw. zum jeweiligen pharmazeutisch verträglichen Salz oder Ester von S-Flurbiprofen von größer oder gleich 60:40 vorliegt. Besonders bevorzugt ist es, wenn das Molverhältnis größer oder gleich 95:5 ist, wobei ein Molverhältnis ab 95:5 "im wesentlichen enantiomerenrein" im Sinne der Erfindung ist. Insbesondere bevorzugt ist es, wenn das Molverhältnis größer oder gleich 98:2, ≥ 99,5:0,5 oder ≥ 99,9:0,1 ist, wobei ein Molverhältnis ab 98:2 "enantiomerenrein" im Sinne der Erfindung ist.

Die gemäß der erfindungsgemäßen Verwendung hergestellten Arzneimittel und Arzneimittelzusammensetzungen bzw. die Arzneimittel und Arzneimittelzusammensetzungen zur erfindungsgemäßen Verwendung enthalten Tarenflurbil bzw. sein pharmazeutisch verträgliches Salz oder Ester vorzugsweise in den genannten geeigneten Formen enantiomerer Reinheit oder Anreicherung.

Wenn technologisch bei der Herstellung des Wirkstoffes Tarenflurbil vertretbar, sollte reines Tarenflurbil bzw. dessen pharmazeutisch verträgliches Salz oder Ester, in dem sich mit modernen analytischen Methoden kein S-Flurbiprofen bzw. entsprechendes S-Flurbiprofensalz oder -Ester nachweisen lässt, in den erfindungsgemäßen Verfahren bzw. zur Herstellung der erfindungsgemäßen Arzneimittel bzw. Arzneimittelzusammensetzungen Verwendung finden.

Tarenflurbil zeigt im Gegensatz zu den meisten anderen R-NSAIDs keine bzw. allenfalls eine äußerst geringe Inversion von der R-Form in die S-Form in vivo, wenn es an Menschen appliziert wird. Deshalb besteht auch nach Gabe hoher therapeutischer Dosen kein Risiko, dass durch Inversion von Tarenflurbil zu S-Flurbiprofen toxische Konzentrationen von S-Flurbiprofen im menschlichen Organismus auftreten können. Wegen der hohen enantiomeren Stabilität von Tarenflurbil in vivo beim Menschen sollten Arzneimittel mit möglichst weitgehend enantiomer reinem Wirkstoff hergestellt werden, um ein besonders günstiges Nutzen-Risiko Verhältnis bei der erfindungsgemäßen Behandlung schmerzhafter Neuropathien oder neuropathischer Schmerzen erzielen zu können. Enantiomerenreines oder im wesentlichen enantiomerenreines Tarenflurbil ist mehreren kommerziellen Wirkstoffherstellern nach den internationalen Good Manufacturing Practices (GMP) für pharmazeutische Wirkstoffe hergestellt erhältlich.

Sämtliche in der vorliegenden Beschreibung der Erfindung und den Ansprüchen aufgeführten Angaben zu geeigneten Tagesdosen, Einzeldosen und Wirkstoffkonzentrationen beziehen sich auf Tarenflurbil bzw. sein jeweiliges pharmazeutisch verträgliches Salz oder Ester einschließlich des Anteils an S-Flurbiprofen bzw. S-Enantiomer des jeweiligen pharmazeutisch verträglichen Salzes oder Esters, der gemäß dem Grad der Enantiomerenreinheit oder der Anreicherung der eingesetzten Form von Tarenflurbil bzw. seines pharmazeutisch verträglichen Salzes oder Esters gegebenenfalls zusätzlich vorliegt.

Wegen der geringen Toxizität kann die verabreichte Tagesdosis in weiten Grenzen den individuellen Gegebenheiten des Patienten angepasst werden. Bei systemischer Anwendung, wenn also die gegebene Dosis über den Blutkreislauf im Organismus verfügbar gemacht werden soll, sollte die Tagesdosis bei mindestens 1 mg/kg Körpergewicht liegen und kann bis 50 mg/kg oder höher gesteigert werden. Eine bevorzugte Dosierung für die systemische Anwendung ist eine Tagesdosis von 2 bis 30 mg/kg Körpergewicht, besonders bevorzugt 3 bis 25 mg/kg Körpergewicht, insbesondere bevorzugt 5 bis 20 mg/kg Körpergewicht und am meisten bevorzugt 10 bis 20 mg/kg Körpergewicht.

Die Tagesdosis sollte, abhängig von der Freisetzungskinetik der pharmazeutischen Zubereitung und der Applikationsform, in ein bis fünf, vorzugsweise ein bis vier Einheiten aufgeteilt werden, so dass eine Applikation von einmal täglich bis fünfaml, vorzugsweise viermal täglich erfolgt.

Für die systemische Anwendung kommen alle bekannten Applikationsrouten wie oral, peroral, intramuskulär, intravenös, intraperitoneal, buccal, sublingual, nasal, transdermal, per Inhalationem und rektal in Frage, für welche die bekannten pharmazeutischen Formulierungen eingesetzt werden können.

Orale oder rektale Formulierungen, die in fester Form verabreicht werden, enthalten vorzugsweise als Einzeldosisform 30 mg bis 1800 mg, bevorzugt 50 mg bis 1200 mg, besonders bevorzugt 100-1000 mg und ganz besonders bevorzugt 200-800 mg Tarenflurbil bzw. ein pharmazeutisch verträgliches Salz oder Ester davon. Für Suppositorien werden die Einzeldosen ähnlich aufgeteilt wie bei den festen oralen Formulierungen.

Lösungen oder Suspensionen für die parenterale Gabe enthalten die gesamte Tagesdosis bevorzugt in einer Einzeldosisform, können jedoch je nach dem therapeutischen Bedarf auch als niedriger dosierte Einzeldosisformen für die mehrmalige tägliche Anwendung zubereitet werden.

Bei oralen Trinkzubereitungen kann die gesamte Tagesdosis, auch wenn sie 1800 mg überschreitet, auf einmal eingenommen werden. Lösungen oder Suspensionen für die orale Applikation können die für einen typischen Therapiezeitraum, wie z.B. eine oder mehrere Wochen oder einen oder mehrere Monate, nötige Menge enthalten, wobei dann eine Tagesdosis oder eine Einzeldosis durch Abmessen einer Teilmenge erhalten wird.

Neben der systemischen Anwendung kann Tarenflurbil bzw. ein pharmazeutisch verträgliches Salz oder Ester davon zur lokalen Therapie schmerzhafter Neuropathien oder neuropathischer Schmerzen eingesetzt werden. Dazu wird eine topisch zu verabreichende pharmazeutische Formulierung auf die Haut der erkrankten Körperstelle appliziert. Für diese Applikationsroute eignen sich alle bei Topika bekannten pharmazeutischen Formulierungen, wie z.B. Salben, Cremes, Emulsionen, Gele, Pflaster.

Für die topische Anwendungen eignen sich Formulierungen mit Konzentrationen an Tarenflurbil oder seinem pharamzeutisch verträglichen Salz oder Ester von 0,5 g/100 g bis 25 g/100 g Zubereitung, bevorzugt von 1 g/100 g bis 20 g/100 g Zubereitung, besonders bevorzugt von 1 g/100 g bis 15 g/100 g Zubereitung, insbesondere bevorzugt von 1,5 g/100 g bis 10 g/100 g Zubereitung und am meisten bevorzugt von 5 g/100 g bis 10 g/100 g Zubereitung.

Die pharmazeutische Formulierung der efindungsgemäßen Arzneimittel bzw. Arzneimittelzusammensetzungen kann in jeder gewünschten und in der pharmazeutischen Technologie bekannten Form hergestellt werden. Hierzu gehören z.B. Tabletten, Kapseln, Dragees, Granulate, unsterile Lösungen und Suspensionen für die orale Applikation, Nanopellets, sterile Lösungen und Suspensionen für die parenterale Gabe, Suppositorien, Aerosole, Salben, Cremes, Emulsionen und Liposomenzubereitungen.

Die gemäß der Erfindung brauchbaren oralen pharmazeutischen Zubereitungen und intramuskulär oder intraperitoneal zu verabreichenden Zubereitungen können sowohl in schnell freisetzender, als auch in modifiziert freisetzender Formulierung hergestellt und therapeutisch verwendet werden. Insbesondere bei den oralen Zubereitungen bietet es sich an, zur Verminderung der Einnahmehäufigkeit, zur Verbesserung der Compliance des Patienten und zur Verbesserung der Verträglichkeit verzögert freisetzende pharmazeutische Formulierungen zur Verfügung zu stellen. Die Freisetzung kann kontinuierlich oder pulsatil über mehrere zeitlich versetzte Einzelfreisetzungen gesteuert werden. Zur Reduzierung der Applikationshäufigkeit bei intramuskulär oder intraperitoneal anzuwendenden Formulierungen können die in der pharmazeutischen Technologie bekannten Zubereitungen zur verzögerten Wirkstoffabgabe wie z.B. Kristallsuspensionen und biodegradable Hilfsstoffe eingesetzt werden.

Gemäß einer bevorzugten Ausführungsform der Erfindung wird das Tarenflurbil oder dessen pharmazeutisch verträgliches Salz oder Ester im Rahmen der erfindungsgemäßen Verfahren, Verwendungen und Arzneimittel bzw. Arzneimittelzusammensetzungen über einen längeren Zeitraum angewandt bzw. verabreicht, vorzugsweise über mehrere Wochen oder mehrere Monate, z. B. über mehr als 3 Monate, mehr als 6 Monate, oder mehr als 12 Monate.

Erfindungsgemäße Arzneimittel bzw. Arzneimittelzusammensetzungen können neben Tarenflurbil oder dessen pharmazeutisch verträglichem Salz oder Ester als Wirkstoff gegebenenfalls weitere Wirkstoffe sowie einen oder mehrere pharmazeutisch verträgliche Hilfsstoffe umfassen.

Der Begriff pharmazeutisch verträgliche Derivate bezieht sich auf Derivate hergestellt aus Tarenflurbil und pharmazeutisch verträglichen anorganischen oder organischen Basen zur Salzbildung, alkoholischen Verbindungen zur Esterbildung.
Hierzu zählen insbesondere Metallsalze, z.B. mit Aluminium, Calcium, Lithium, Magnesium, Kalium, Natrium und Zink oder ogranische Salze, z.B. mit Lysin, N,N'-Dibenzylethylendiamin, Cholin, Diethanolamin, Ethylendiamin, Meglumin, Trometamin, Arginin und Alkylaminen mit 1 bis 6 C-Atomen oder Ester, z.B. mit aliphatischen oder isoaliphatischen Alkoholen mit 1 bis 8 C-Atomen. Tarenflurbil kann im Rahmen der Erfindung auch als freie Säure eingesetzt werden.

Gemäß der Erfindung kann das Tarenflurbil bzw. das pharmazeutisch verträgliche Salz oder Ester davon in vorteilhafter Weise in freier oder fixer Kombination mit anderen für die Behandlung schmerzhafter Neuropathien oder neuropathischer Schmerzen geeigneten Wirkstoffen verabreicht werden.

Für die medikamentöse Kombinationstherapie schmerzhafter Neuropathien oder neuropathischer Schmerzen kann Tarenflurbil bzw. ein pharmazeutisch verträgliches Salz oder Ester davon in fixen Kombinationen mit anderen, bei dieser Indikation wirksamen Substanzen eingesetzt werden. Für die systemische Kombinationstherapie kommen bevorzugt Substanzen aus den Wirkstoffgruppen Antidepressiva, Antikonvulsiva, Opioide, Cannabinoide und Myotonolytika in Frage. Für die topische Kombinationstherapie können Kombinationen aus Tarenflurbil und topischen Analgetika oder Lokalanästhetika eingesetzt werden. Bei der Herstellung von Arzneimitteln mit Fixkombinationen aus Tarenflurbil bzw. aus einem pharmazeutisch verträglichen Salz oder Ester davon und anderen Wirkstoffen werden die Dosen so gewählt, dass Tarenflurbil (bzw. das verwendete pharmazeutisch verträgliche Salz oder Ester davon) in den oben beschriebenen Dosen und der Kombinationswirkstoff in der therapeutisch üblichen Dosis für den betreffenden Einzelwirkstoff eingesetzt wird.

Erfindungsgemäß eignet sich Tarenflurbil somit nicht nur zur Therapie schmerzhafter Neuropathien, sondern auch zur Behandlung von Schmerzen, die sich aufgrund der vorliegenden Schädigung aus schmerzhaften Neuropathien und Nozizeptorschmerzen zusammensetzen. Diese gemischten Schmerzen kommen vor, wenn in eng umschriebenen Körperarealen gleichzeitig schmerzende Gewebetraumen und schmerzende periphere oder zentrale Nervenschädigungen vorliegen. Gerade dieser Schmerztyp wird in der Praxis häufig nicht adäquat behandelt, weil die bekannten Nicht-Opioid-Analgetika nur bei Nozizeptorschmerzen, aber nicht bei schmerzhaften Neuropathien wirksam werden, während die meisten zur Behandlung schmerzhafter Neuropathien eingesetzten Wirkstoffe nur bei diesem Schmerztyp, aber nicht bei Nozizeptorschmerzen wirksam sind. Bei gemischten Schmerzen wird Tarenflurbil bzw. ein pharmazeutisch verträgliches Salz oder Ester davon sowohl über die bekannte schmerzlindernde Wirkung bei Nozizeptorschmerzen, als auch über die erfindungsgemäß gefundene Wirkung bei schmerzhaften Neuropathien gleichzeitig wirksam. Tarenflurbil und seine pharmazeutisch verträglichen Salze und Ester verfügen daher über ein bekanntes nozizeptives sowie ein zuvor unbekanntes, neuropathisches Schmerzpotenzial ähnlich den opioiden Wirksubstanzen, jedoch ohne deren bekanntes Suchtpotenzial und atemdepressives Potential. Diese Schmerzkombination ist bisher für keine andere nicht-opioide Substanz beschrieben und stellt daher einen hohen bisher nicht bekannten therapeutischen Nutzen dar. Wegen seiner ausgezeichneten Verträglichkeit kann Tarenflurbil (bzw. dessen pharmazeutisch verträglichen Salze und Ester) bei gemischten Schmerzen von Anfang an in hoher Dosis eingesetzt werden. Ein vergleichbar breites Wirkspektrum bei schmerzhaften Neuropathien und bei Nozizeptorschmerzen weisen allenfalls die Opioidanalgetika auf, die jedoch wegen ihres Nebenwirkungsprofils nur bei strenger Indikationsstellung eingesetzt werden sollen. Häufig vorkommende gemischte Schmerzen sind Rückenschmerzen und Tumorschmerzen, für deren Behandlung Tarenflurbil (bzw. dessen pharmazeutisch verträglichen Salze und Ester) unabhängig von einer Differentialdiagnose ein zu bevorzugendes Arzneimittel wäre.

Als weitere Wirkstoffe, die kombiniert mit Tarenflurbil (bzw. dessen pharmazeutisch verträglichen Salzen oder Ester) z.B. als systemisch anzuwendende Arzneimittel hergestellt und als fixe Kombination medizinisch verwendet werden können, eignen sich alle zur Therapie schmerzhafter Neuropathien eingesetzten Wirkstoffe wie z.B. Antidepressiva (Amitriptylin, Nortriptylin; Desipramin, Maprotilin, Venlafaxin, Duloxetin, Bupropion), Antikonvulsiva (Carbamazepin, Oxcarbazepin, Lamotrigin, Gabapentin, Pregabalin), Opioide (Tramadol, Morphin, Oxycodon), Cannabinoide (Tetrahydro-Cannabinol), Myotonolytika (Baclofen), NMDA-Antagonisten (Dextromethorphan, Ketamin, Memantine), oder Radikalfänger (Alpha-Liponsäure). Für topisch anzuwendende Arzneimittel kommen als Kombinationswirkstoffe für Tarenflurbil bzw. dessen pharmazeutisch verträglichen Salzen oder Derivaten Lokalanästhetika (z.B. Capsaicin, Lidocain und Benzocain) in Frage.

Unter die pharmazeutisch verträglichen Hilfsstoffe sind gemäß der Erfindung je nach Formulierung Trägerstoffe wie Stärke, Zucker, mikrokristalline Zellulose, Verdünner, Granulierhilfsmittel, Gleitmittel, Bindemittel, Zerfallsförderer und ähnliches zu verstehen. Besonders vorteilhafte pharmazeutische Formulierungen für Tarenflurbil enthaltende Arzneimittel sind in EP 0 607 128, EP 0 641 200 und EP 0 615 440 beschrieben.
Die folgenden Beispiel sollen die Erfindung näher veranschaulichen, ohne sie jedoch auf die konkret beschriebenen Ausführungsformen zu beschränken. Soweit nichts anderes angegeben ist, beziehen sich sämtliche Angaben von Teilen und % im Rahmen der vorliegenden Erfindung auf das Gewicht bzw. das Gesamtgewicht der Zusammensetzung/Mischung.

### Beispiel 1

Als Tiermodelle, die wesentliche Elemente der klinischen Schmerzsyndrome bei Neuropathien zeigen, wurden das Chronic Constriction Injury-Modell (CCI Modell) [Bennet GJ, Xie YK: A peripheral mononeuropathy in rat that produces disorders of pain sensation like those seen in man. Pain 1988, 33:87-107] und das Spared Nerve Injury-Modell (SNI Model) [Decosterd I, Woolf CJ: Spared Nerve injury: an animal model of persistent peripherel neuropathic pain. Pain 2000, 87:149-158] angewandt. Bei beiden Modellen wird den Versuchstieren operativ eine definierte Nervenschädigung zugefügt. Anhand des Schmerzverhaltens der Tiere kann die Wirksamkeit von Substanzen bei schmerzhaften Neuropathien quantitativ gemessen werden. Die Untersuchungen wurden gemäß den publizierten Modellen an Ratten durchgeführt. Bei Ratten kann die ausschließliche Wirkung von Tarenflurbil gemessen werden, weil sie, wie der Mensch, Tarenflurbil praktisch nicht zu S-Flurbiprofen invertieren. Dies trifft für die meisten anderen Labortierspezies nicht zu.

In einer ersten Versuchsserie wurde den Versuchstieren operativ eine Nervenläsion nach dem SNI Modell sowie nach dem CCI Modell zugefügt. Ab dem 10. Tag bis zum 21. Tag nach der Operation erhielten Gruppen von jeweils 12 Tieren zweimal täglich intraperitoneal die Testsubstanzen verabreicht. Beim Testen nach dem SNI Modell wurden pro Applikation, jeweils pro kg Körpergewicht, 2,5; 4,5 oder 9 mg Tarenflurbil (als RF2.5, RF4.5 und RF9 bezeichnet), 4,5 mg S-Flurbiprofen (SF4.5), reines Vehikel (Vehikel - als Kontrolle) oder 25 mg Gabapentin (Gaba25 - als Referenzsubstanz) verabreicht. Gemessen wurden die für schmerzhafte Neuropathien typischen Verhaltensparameter, nämlich die mechanische Allodynie mit dem von Frey Aestesiometer, die Kälteallodynie mit dem Acetontest und die Kältehyperalgesie mit der 2°C kalten Platte. Beim Testen nach dem CCI Modell wurden pro Gruppe 4,5 oder 9 mg/kg Körpergewicht Tarenflurbil (Rf4.5 und RF9), 9mg/kg Körpergweicht S-Flurbiprofen (SF9) oder Vehikel (Vehikel) verabreicht. Gemessen wurde ebenfalls die mechanisch/statische Allodynie mit dem von Frey Haar Test, die Kälteallodynie mit dem Acetontest und die Kältehyperalgesie mit der 2°C kalten Platte

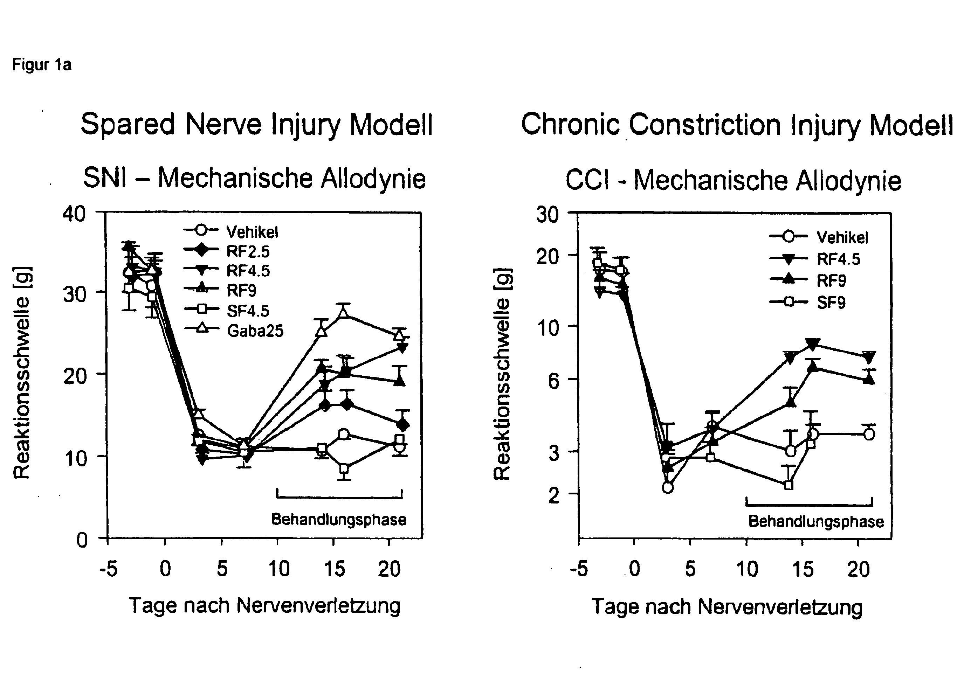

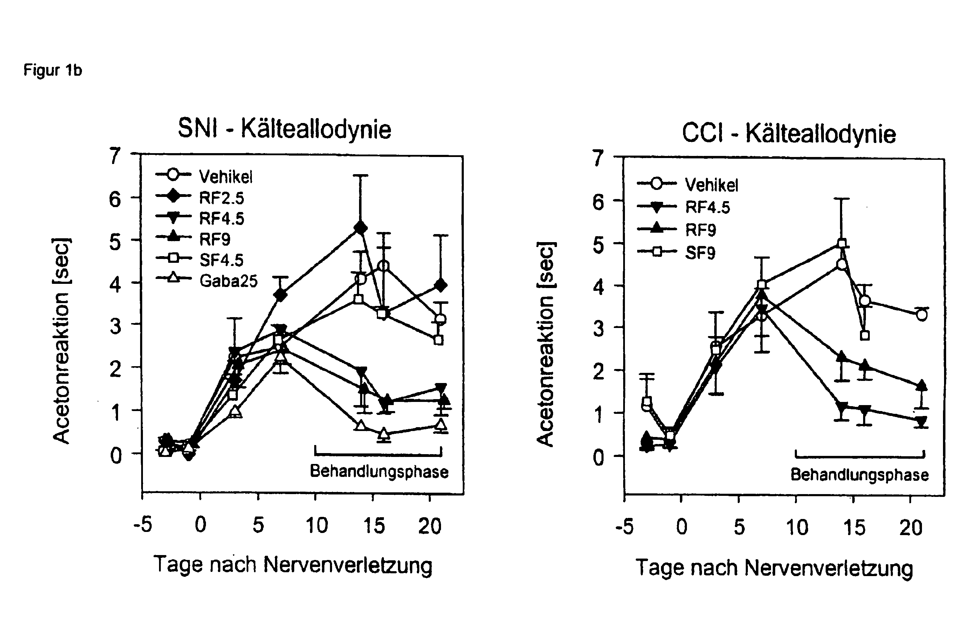

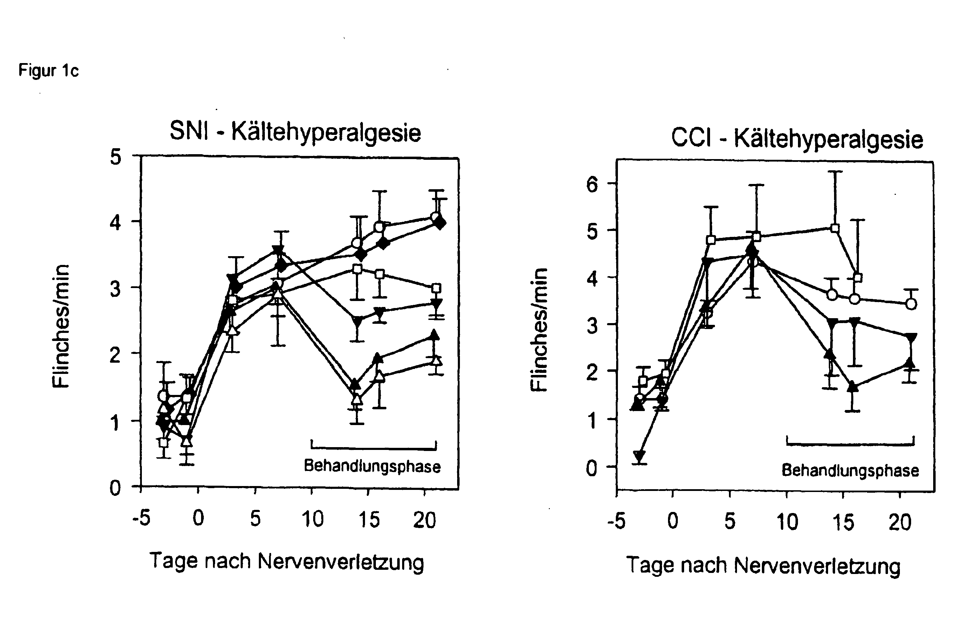

Die Ergebnisse aus den Versuchsserien sind in Figur 1a, 1b und 1c gezeigt. Eine signifikante Reduktion der Allodynie wurde erreicht mit 4,5 und 9 mg/kg Tarenflurbil zweimal täglich. S-Flurbiprofen zeigte keinen signifikanten Effekt. Die Testung von höheren Dosen als 4,5 mg/kg S-Flurbiprofen zweimal täglich war wegen gastrointestinaler Blutungen ab etwa 1 Woche Behandlungsdauer bei einzelnen Tieren nicht möglich. Das bei Neuropathien eingesetzte Gabapentin als Positivkontrolle zeigte keinen signifikanten Unterschied zu Tarenflurbil.

### Beispiel 2

In einer weiteren Versuchsserie wurden die Tiere nach einer Nervenschädigung gemäß dem SNI Modell bereits ab dem ersten postoperativen Tag über zwei Wochen mit zweimal täglich 9 mg/kg Körpergewicht Tarenflurbil oder Vehikel intraperitoneal behandelt. Gemessen wurde die mechanisch dynamische Allodynie mittels des von Frey Aesthesiometers sowohl auf der ipsilateralen als auch auf der kontralateralen Seite sowie die Kälteallodynie mit der 10°C kalten Platte nur ipsilateral.

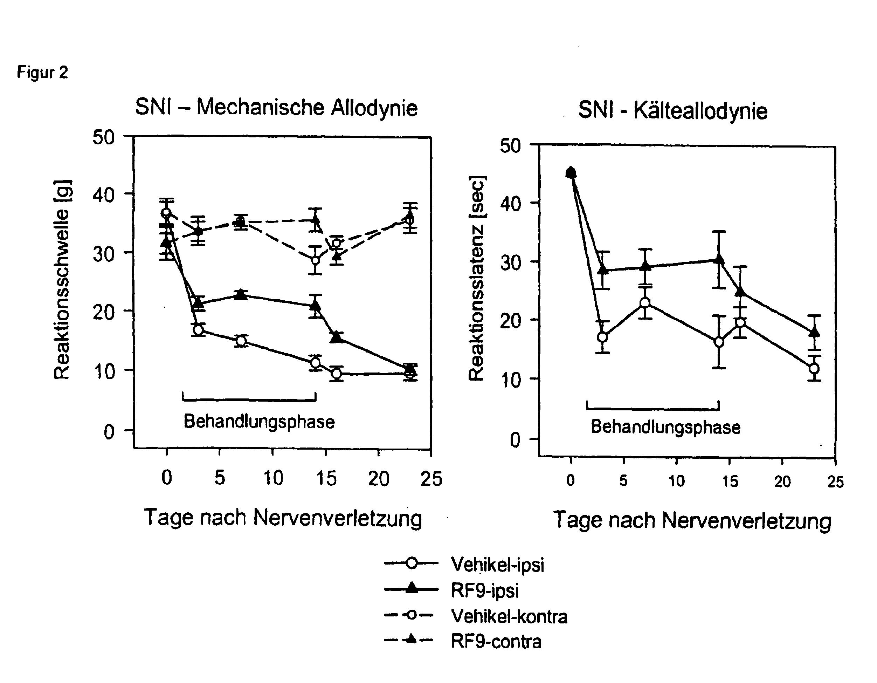

Die Ergebnisse dieser Versuchsserie sind in Figur 2 zusammengestellt. Tarenflurbil (RF9-ipsi) zeigt bei der mechanischen Allodynie und bei der Kälteallodynie während der gesamten zweiwöchigen Behandlungsdauer einen signifikant unterschiedlichen Effekt im Vergleich zum Vehikel (Vehikel-ipsi) auf der ipsilateralen Seite. Auf der kontralateralen Seite unterscheiden sich Tarenflurbil (RF9-contra) und Vehikel (Vehikel-kontra) nicht. Nach Beendigung der Behandlung nimmt der schmerzlindernde Effekt bei der mit Tarenflurbil behandelten Gruppe langsam ab und erreicht acht Tage nach Behandlungsende die Schmerzintensität der Vehikelgruppe.

### Beispiel 3

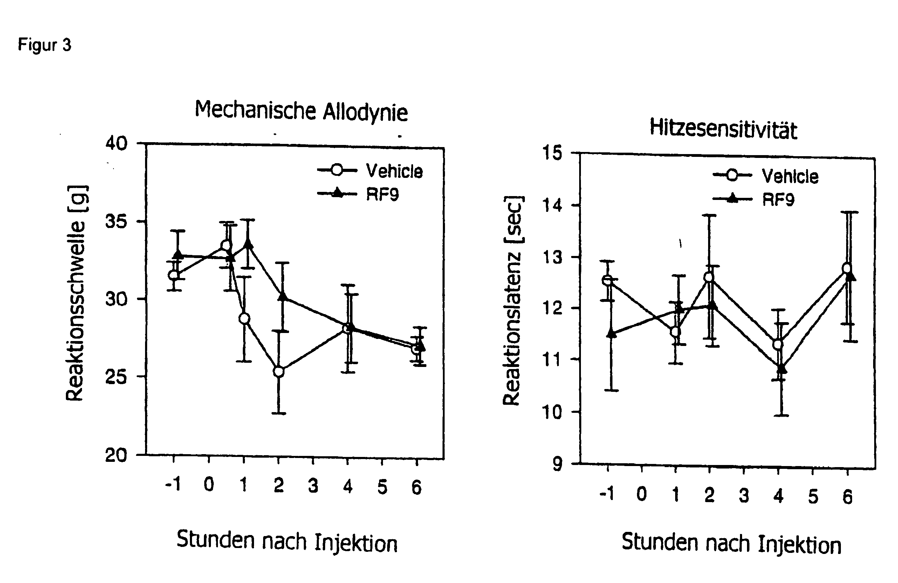

Zur Qualifizierung der oben genannten Versuchsserien wurden in einer weiteren Versuchsserie an nicht geschädigten Ratten einmalig 9 mg/kg Körpergewicht Tarenflurbil (RF9) oder Vehikel (Vehicle) intraperitoneal appliziert und bis 6 Stunden nach der Applikation die mechanische Allodynie mit dem von Frey Aesthesiometer sowie die die Hitzesensitivität nach dem Hargreaves Modell gemessen. Die in Figur 3 dargestellten Ergebnisse zeigen keine signifikanten Unterschiede zwischen der mit Tarenflurbil und der mit Vehikel behandelten Gruppe. Das heißt, dass die in den Beispielen 1 und 2 für Tarenflurbil gemessenen Effekte ausschließlich auf die Wirkung bei schmerzhaften Neuropathien zurückzuführen sind.

## Patentansprüche

1. Tarenflurbil bzw. ein pharmazeutisch verträgliches Salz oder ein Ester mit aliphatischen oder isoaliphatischen Alkoholen mit 1-8 C-Atomen davon, in gegenüber Flurbiprofen-Racemat bzw. einem Racemat des genannten Salzes oder Esters angereicherter Form zur Anwendung in der Behandlung von
a) schmerzhafter Neuropathie
b) schmerzhafter Neuropathie, die gleichzeitig von nozizeptiven Schmerzzuständen begleitet wird
c) peripherem bzw. vorwiegend peripherem neuropathischem Schmerz; oder
d) zentralem bzw. vorwiegend zentralem neuropathischem Schmerz.

2. Verbindung zur Anwendung nach Anspruch 1, wobei es sich bei dem vorwiegend peripheren neuropathischen Schmerz um solchen handelt, der aus folgenden neuropathischen Schmerzen ausgewählt ist bzw. eine Ursache hat, die aus der Gruppe folgender Ursachen ausgewählt ist:
- Systemische Erkrankungen, z.B. diabetische Neuropathie;
- Arzneimittel-induzierte Läsionen, z.B. Neuropathie aufgrund von Chemotherapie:
- Traumatisches Syndrom und Entrapment-Syndrom;
- Läsionen der Nervenwurzeln und der Posteriorganglien;
- Neuropathien nach HIV Infektionen;
- Neuralgien nach Herpesinfektionen;
- Nervenwurzelavulsionen;
- Läsionen der Cranialnerven;
- Craniale Neuralgien, z.B. Trigeminalneuralgie;
- neuropathischer Krebsschmerz;
- Phantomschmerz;
- Kompression der peripheren Nerven, Nerven-Plexus und Nervenwurzeln;
- paraneoplastische periphere Neuropathie und Ganglionopathie;
- Komplikationen von Krebstherapien, z.B. Chemotherapie, Bestrahlung und chirurgische Eingriffe;
- komplexes regionales Schmerzsyndrom
- Läsionen des Typs I (vormals bekannt als sympathische Reflexdystrophie); und
- Läsionen des Typs II (in etwa der Causalgie entsprechend), oder wobei es sich bei dem vorwiegend zentralen neuropathischen Schmerz um solchen mit einer Ursache handelt, die ausgewählt ist aus der folgenden Gruppe von Ursachen:
- Zerebrale Läsionen, die vorwiegend thalamisch sind;
- Infarkt, z.B. thalamischer Infarkt oder Hirnstamminfarkt;
- zerebrale Tumore oder Abszesse, die den Thalamus oder den Hirnstamm komprimieren;
- Multiple Sklerose;
- Hirnoperationen, z.B. Thalamotomie bei motorischen Störungen;
- Läsionen des Rückenmarks;
- Verletzungen des Rückenmarks;
- Operationen am Rückenmark, z.B. anterolaterale Cordotomie;
- ischämische Läsionen;
- Anteriore Spinalarterie-Syndrom;
- Wallenbergs Syndrom; und
- Syringomyelie.

3. Verbindung zur Anwendung nach Anspruch 1 oder 2, wobei das Tarenflurbil bzw. sein pharmazeutisch verträgliches Salz oder Ester in einem Molverhältnis zu S-Flurbiprofen bzw. zum jeweiligen pharmazeutisch verträglichen Salz oder Ester von S-Flurbiprofen von größer oder gleich 60:40, vorzugsweise größer oder gleich 95:5, besonders bevorzugt größer oder gleich 98:2, ganz besonders bevorzugt größer oder gleich 99,5:0,5 und am meisten bevorzugt größer oder gleich 99,9:0,1 vorliegt.

4. Verbindung zur Anwendung nach einem der vorstehenden Ansprüche, wobei das Tarenflurbil und das gegebenenfalls vorhandene S-Flurbiprofen als freie Säure, als Salz mit anorganischen oder organischen Salzbildnern, als Komplex mit anorganischen oder organischen Komplexbildnern, oder als Säureester vorliegt.

5. Verbindung zur Anwendung nach einem der vorstehenden Ansprüche, wobei das Tarenflurbil bzw. sein pharmazeutisch verträgliches Salz oder Ester für die systemische Anwendung in einer Tagesdosis von mindestens 1 mg/kg Körpergewicht bis 50 mg/kg Körpergewicht oder darüber, bevorzugt in Tagesdosen von 2 mg/kg bis 30 mg/kg Körpergewicht, besonders bevorzugt von 3 bis 25 mg/kg Körpergewicht, ganz besonders bevorzugt von 5 bis 20 mg/kg Körpergewicht, und am meisten bevorzugt von 10 bis 20 mg/kg Körpergewicht eingesetzt wird.

6. Verbindung zur Anwendung nach einem der Ansprüche 1 bis 5, wobei die Tagesdosis als einmalige Dosis oder in mehreren Einzeldosen verabreicht wird.

7. Verbindung zur Anwendung nach einem der Ansprüche 1 bis 6, wobei für oral oder rektal als Ganzes zu applizierende Formulierungen Einzeldosisformen mit 30 mg bis 1800 mg Wirkstoffgehalt, bevorzugt mit 50 mg bis 1200 mg, besonders bevorzugt mit 100 bis 1000 mg und ganz besonders bevorzugt mit 200 bis 800 mg Wirkstoffgehalt und für trinkbare, orale Formen sowie Injektionsformen Einzeldosisformen von minimal 30 mg bis zur maximalen Tagesdosis hergestellt bzw. verabreicht werden.

8. Verbindung zur Anwendung nach einem der Ansprüche 1 bis 7, wobei das Arzneimittel über einen längeren Zeitraum angewandt wird, vorzugsweise über mehrere Wochen oder Monate.

9. Verbindung zur Anwendung nach einem der Ansprüche 1 bis 8, wobei das Tarenflurbil bzw. sein pharmazeutisch verträgliches Salz oder Ester mit einem oder mehreren zur systemischen Behandlung neuropathischer Schmerzen geeigneten Wirkstoffen in der üblichen therapeutischen Dosis kombiniert wird, z.B. mit Antidepressiva (Amitriptylin, Nortriptylin; Desipramin, Maprotilin, Venlafaxin, Duloxetin, Bupropion), Antikonvulsiva (Carbamazepin, Oxcarbazepin, Lamotrigin, Gabapentin, Pregabalin), Opioide (Tramadol, Morphin, Oxycodon), Cannabinoide (Tetrahydro-Cannabinol), Myotonolytika (Baclofen), NMDA-Antagonisten (Dextromethorphan, Ketamin, Memantine), Radikalfänger (Alpha-Liponsäure).

10. Verbindung zur Anwendung nach einem der Ansprüche 1 bis 9, wobei der Wirkstoff/die Wirkstoffe aus den Arzneimitteln schnell freigesetzt oder modifiziert, z.B. retardiert oder pulsatil, freigesetzt wird/werden.

11. Verbindung zur Anwendung nach einem der Ansprüche 1 bis 10, wobei die systemische Applikation oral, peroral, intramuskulär, intravenös, intraperitoneal, buccal, nasal, transdermal, per Inhalationem oder rektal erfolgt.

12. Verbindung zur Anwendung nach einem der Ansprüche 1 bis 11, wobei die Applikation oral erfolgt und das Arzneimittel in Form von Tabletten, Kapseln, Dragees, Granulat, einer unsterilen Lösung oder einer Suspension vorliegt oder die Applikation parenteral erfolgt und das Arzneimittel in Form einer sterilen Lösung oder Suspension vorliegt oder die Applikation rektal erfolgt und das Arzneimittel in Form von Suppositorien vorliegt oder die Applikation oral oder nasal erfolgt und das Arzneimittel in Form eines Aerosols vorliegt.

13. Verbindung zur Anwendung nach einem der Ansprüche 1 bis 12, wobei die Applikation topisch erfolgt und die Wirkstoffkonzentration für die lokale Anwendung in Form von Topika weniger als 0,5 g/100 g bis 25 g/100 g Zubereitung oder darüber, bevorzugt von 1 g/100 g bis 20 g/100 g Zubereitung, besonders bevorzugt von 1 g/100 g bis 15 g/100 g Zubereitung, ganz besonders bevorzugt von 1,5 g/100 g bis 10 g/100 g Zubereitung und am meisten bevorzugt von 5 g/100 g bis 10 g/100 g Zubereitung beträgt.

14. Verbindung zur Anwendung nach Anspruch 13, wobei das Tarenflurbil bzw. sein pharmazeutisch verträgliches Salz oder Ester mit einem oder mehreren zur topischen Behandlung schmerzhafter Neuropathien geeigneten Wirkstoffen in der üblichen therapeutischen Konzentration kombiniert wird, z.B. mit Capsaicin, Lidocain oder Benzocain.

15. Verbindung zur Anwendung nach Anspruch 13 oder 14, wobei das Arzneimittel in Form einer für die topische Gabe auf die Haut geeigneten Formulierung vorliegt, z.B. einer Salbe, einer Creme, eines Gels, einer Lösung, einer Liposomenzubereitung, eines Pflasters, oder eines beschichteten Verbands.

## Claims

1. Tarenflurbil and/or a pharmaceutically tolerable salt or ester with an aliphatic or isoaliphatic alcohol with 1 - 8 C atoms thereof in a form that is enriched with respect to flurbiprofen racemate and/or a racemate of said salt or ester, for use in the treatment of
a) pain-associated neuropathy
b) pain-associated neuropathy that is simultaneously accompanied by states of nociceptive pain
c) peripheral and/or predominantly peripheral neuropathic pain; or
d) central and/or predominantly central neuropathic pain.

2. Compound for use according to claim 1, whereby the predominantly peripheral neuropathic pain is of a type that is selected from the following types of neuropathic pain and/or has a cause that is selected from the group of the following causes:
- systemic diseases, e.g. diabetic neuropathy;
- drug-induced lesions, e.g. neuropathy due to chemotherapy;
- traumatic syndrome and entrapment syndrome;
- lesions in nerve roots and posterior ganglia;
- neuropathies after HIV infections;
- neuralgia after Herpes infections;
- nerve root avulsions;
- cranial nerve lesions;
- cranial neuralgias, e.g., trigeminal neuralgia;
- neuropathic cancer pain;
- phantom pain;
- compression of peripheral nerves, neuroplexus and nerve roots;
- paraneoplastic peripheral neuropathy and ganglionopathy;
- complications of cancer therapies, e.g. chemotherapy, irradiation, and surgical interventions;
- complex regional pain syndrome;
- type I lesions (previously known as sympathetic reflex dystrophy); and
- type II lesions (corresponding approximately to causalgia) or whereby the predominantly central neuropathic pain is of a type that has a cause that is selected from the following group of causes:
- cerebral lesions that are predominantly thalamic;
- infarction, e.g. thalamic infarction or brain stem infarction;
- cerebral tumors or abscesses compressing the thalamus or brain stem;
- multiple sclerosis;
- brain operations, e.g. thalamotomy in cases of motoric disorders;
- spinal cord lesions;
- spinal cord injuries;
- spinal cord operations, e.g. anterolateral cordotomy;
- ischemic lesions;
- anterior spinal artery syndrome;
- Wallenberg's syndrome; and
- syringomyelia.

3. Compound for use according to claim 1 or 2, whereby tarenflurbil and/or its pharmaceutically tolerable salt or ester is present at a molar ratio of larger than or equal to 60:40, preferably larger than or equal to 95:5, particularly preferably larger than or equal to 98:2, more particularly preferably larger than or equal to 99.5:0.5 and most preferably larger than or equal to 99.9:0.1 with respect to S-flurbiprofen and/or the corresponding pharmaceutically tolerable salt or ester of S-flurbiprofen.

4. Compound for use according to any one of the preceding claims, whereby tarenflurbil and S-flurbiprofen, if present, are present as the free acid, as salt with inorganic or organic salt-forming agents, as complex with inorganic or organic complex-forming agents, as acid ester or as acid amide.

5. Compound for use according to any one of the preceding claims, whereby, for systemic application, tarenflurbil and/or its pharmaceutically tolerable salt or ester is used at a daily dose of at least 1 mg/kg body weight to 50 mg/kg body weight or higher, preferably at daily doses of 2 mg/kg to 30 mg/kg body weight, particularly preferably of 3 to 25 mg/kg body weight, more particularly preferably of 5 to 20 mg/kg body weight, and most preferably of 10 to 20 mg/kg body weight.

6. Compound for use according to any one of the claims 1 to 5, whereby the daily dose is administered as a single dose or in several single doses.

7. Compound for use according to any one of the claims 1 to 6, whereby, for oral or rectal formulations to be applied in a single dose, single dose forms with an agent content of 30 mg to 1800 mg, preferably with an agent content of 50 mg to 1200 mg, particularly preferably of 100 to 1000 mg, and more particularly preferably of 200 to 800 mg of the agent, and for potable, oral forms as well as forms for injection, single dose forms of minimally 30 mg up to the maximal daily dose are produced and/or administered.

8. Compound for use according to any one of the claims 1 to 7, whereby the drug is applied for an extended period of time, preferably over several weeks or months.

9. Compound for use according to any one of the claims 1 to 8, whereby tarenflurbil and/or its pharmaceutically tolerable salt or ester is combined with one or more agents, at the common therapeutic dose, that are well-suited for systematic treatment of neuropathic pain, e.g. antidepressants (e.g. amitriptyline, nortriptyline; desipramine, maprotiline, venlafaxine, duloxetine, bupropion), anticonvulsants (e.g. carbamazepine, oxcarbazepine, lamotrigine, gabapentin, pregabalin), opioids (e.g. tramadol, morphine, oxycodone), cannabinoids (e.g. tetrahydro-cannabinol), myotonolytics (e.g. baclofen), and NMDA antagonists (e.g. dextromethorphan, ketamine, memantine), radical scavengers (e.g. alpha-lipoic acid).

10. Compound for use according to any one of the claims 1 to 9, whereby the agent(s) is/are rapidly-released or modified-released from the drugs, e.g. delayed or in pulses.

11. Compound for use according to any one of the claims 1 to 10, whereby systemic application is performed by the oral, peroral, intramuscular, intravenous, intraperitoneal, buccal, nasal, transdermal, inhalative, or rectal route.

12. Compound for use according to any one of the claims 1 to 11, whereby the application is by the oral route and the drug is provided in the form of tablets, capsules, coated tablets, granulate, a non-sterile solution or a suspension or whereby the application is by the parenteral route and the drug is provided in the form of a sterile solution or suspension or whereby the application is by the rectal route and the drug is provided in the form of suppositories or whereby the application is by the oral or nasal route and the drug is provided in the form of an aerosol.

13. Compound for use according to any one of the claims 1 to 12, whereby the application is by the topical route and the agent concentration for local application in the form of topical agents is less than 0.5 g/100 g to 25 g/100 g of preparation or higher, preferably 1 g/100 g to 20 g/100 g of preparation, particularly preferably 1 g/100 g to 15 g/100 g of preparation, more particularly preferably 1.5 g/100 g to 10 g/100 g of preparation, and most preferably 5 g/100 g to 10 g/100 g of preparation.

14. Compound for use according to claim 13, whereby tarenflurbil and/or its pharmaceutically tolerable salt or ester is combined with one or more agent(s), at the common therapeutic concentration, that is/are well-suited for topical treatment of pain-associated neuropathies, e.g. capsaicin, lidocaine or benzocaine.

15. Compound for use according to claim 13 or 14, whereby the drug is provided in the form of a formulation that is well-suited for topical application to the skin, e.g. an ointment, a crème, a gel, a solution, a liposome preparation, a patch or a coated dressing.

## Revendications

1. Tarenflurbil, respectivement un de ses sels pharmaceutiquement acceptables ou un de ses esters avec des alcools aliphatiques ou isoaliphatiques contenant de 1 à 8 atomes de carbone, sous forme enrichie par rapport au racémate de flurbiprofen, respectivement à un racémate du sel ou de l'ester mentionné, pour une utilisation dans le traitement
a) d'une neuropathie douloureuse ;
b) d'une neuropathie douloureuse qui s'accompagne en même temps d'états de douleurs nociceptives ;
c) d'une douleur neuropathique périphérique, respectivement principalement périphérique ; ou
d) d'une douleur neuropathique centrale, respectivement principalement centrale.

2. Composé pour l'utilisation selon la revendication 1, dans lequel il s'agit, en ce qui concerne la douleur neuropathique principalement périphérique, d'une douleur qui est choisie parmi les douleurs neuropathiques suivantes, respectivement dont la cause est choisie parmi le groupe des causes suivantes :
- des maladies systémiques, par exemple une neuropathie diabétique ;
- des lésions induites par des médicaments, par exemple une neuropathie sur base d'une chimiothérapie ;
- un syndrome traumatique et le syndrome des défilés ;
- des lésions des racines nerveuses et des ganglions postérieurs ;
- des neuropathies après des infections dues au VIH ;
- des névralgies après des infections herpétiques ;
- des avulsions des racines nerveuses ;
- des lésions des nerfs crâniens ;
- des névralgies crâniennes, par exemple la névralgie du trijumeau ;
- une douleur neuropathique d'origine cancéreuse ;
- une douleur fantôme ;
- la compression des nerfs périphériques, du plexus nerveux et des racines nerveuses ;
- une ganglionopathie et une neuropathie périphérique paranéoplasique ;
- des complications de thérapies anticancéreuses, par exemple la chimiothérapie, l'irradiation et des interventions chirurgicales ;
- le syndrome de douleur régionale complexe ;
- des lésions de type I (que l'on désignait autrefois par l'expression « dystrophie réflexe sympathique ») ; et
- des lésions de type II (correspondant par exemple à une causalgie),
ou bien dans lequel il s'agit, en ce qui concerne une douleur neuropathique principalement centrale, d'une douleur dont la cause est choisie parmi le groupe des causes suivantes :
- des lésions cérébrales qui sont principalement de type thalamique ;
- l'infarctus par exemple l'infarctus thalamique ou l'infarctus du tronc cérébral ;
- des tumeurs ou des abcès cérébraux qui compriment le thalamus ou le tronc cérébral ;
- la sclérose en plaques ;
- des opérations cervicales par exemple la thalamotomie dans le cas de troubles moteurs ;
- des lésions de la moëlle épinière ;
- des blessures de la moëlle épinière ;
- des opérations qui visent la moëlle épinière, par exemple une chordotomie antérolatérale ;
- des lésions ischémiques ;
- le syndrome de l'artère spinale antérieure ;
- le syndrome de Wallenberg ; et
- la syringomyélie.

3. Composé pour l'utilisation selon la revendication 1 ou 2, dans lequel le tarenflurbil, respectivement son sel ou son ester pharmaceutiquement acceptable est présent dans un rapport molaire au S-flurbiprofen, respectivement au sel ou à l'ester respectif pharmaceutiquement acceptable du S-flurbiprofen, supérieur ou égal à 60:40, de préférence supérieur ou égal à 95:5, de manière particulièrement préférée supérieur ou égal à 98:2, de manière tout particulièrement préférée supérieur ou égal à 99,5:0,5 et de manière de loin préférée supérieur ou égal à 99,9:0,1.

4. Composé pour l'utilisation selon l'une quelconque des revendications précédentes, dans lequel le tarenflurbil et le S-flurbiprofen présent le cas échéant, sont présents sous la forme de l'acide libre, sous la forme d'un sel avec des agents de salification inorganiques ou organiques, sous la forme d'un complexe avec des agents complexants inorganiques ou organiques, ou sous la forme d'un ester d'acide.

5. Composé pour l'utilisation selon l'une quelconque des revendications précédentes, dans lequel le tarenflurbil, respectivement son sel ou son ester pharmaceutiquement acceptable est mis en oeuvre pour l'application systémique dans une dose quotidienne d'au moins 1 mg/kg de poids du corps à 50 mg/kg de poids du corps ou plus, de préférence dans des doses quotidiennes de 2 mg/kg à 30 mg/kg de poids du corps, de manière particulièrement préférée de 3 à 25 mg/kg de poids du corps, de manière tout particulièrement préférée de 5 à 20 mg/kg de poids du corps, et de manière de loin préférée de 10 à 20 mg/kg de poids du corps.

6. Composé pour l'utilisation selon l'une quelconque des revendications 1 à 5, dans lequel la dose quotidienne est administrée sous la forme d'une dose unique ou sous la forme de plusieurs doses fractionnées.

7. Composé pour l'utilisation selon l'une quelconque des revendications 1 à 6, dans lequel on prépare, respectivement on administre, pour des formulations à appliquer entièrement par voie orale ou par voie rectale, des formes posologiques individuelles possédant une teneur en substance active de 30 mg à 1800 mg, de préférence de 50 mg à 1200 mg, de manière particulièrement préférée de 100 à 1000 mg et de manière tout particulièrement préférée de 200 à 800 mg, et pour des formes orales buvables ainsi que pour des formes d'injection, des formes posologiques individuelles allant d'une dose minimale de 30 mg jusqu'à la dose quotidienne maximale.

8. Composé pour l'utilisation selon l'une quelconque des revendications 1 à 7, dans lequel le médicament est appliqué pendant un laps de temps prolongé, de préférence pendant plusieurs semaines ou mois.

9. Composé pour l'utilisation selon l'une quelconque des revendications 1 à 8, dans lequel le tarenflurbil, respectivement son sel ou son ester pharmaceutiquement acceptable est combiné dans la dose thérapeutique habituelle avec une ou plusieurs substances actives appropriées pour le traitement systémique de douleurs neuropathiques, par exemple avec des antidépresseurs (l'amitriptyline, la nortriptyline, la désipramine, la maprotiline, la venlafaxine, la duloxétine, le bupropion), des anticonvulsifs (la carbamazépine, l'oxcarbazépine, la lamotrigine, la gabapentine, la prégabaline), des opioïdes (le tramadol, la morphine, l'oxycodon), des cannabinoïdes (le tétrahydro-cannabinol), des myotonolytiques (le baclofen), des antagonistes du NMDA (le dextrométhorphane, la cétamine, la mémantine), des fixateurs de radicaux libres (l'acide alpha-lipoïque).

10. Composé pour l'utilisation selon l'une quelconque des revendications 1 à 9, dans lequel la substance active/les substances actives est/sont libérée(s) rapidement ou est/ sont libérée(s) à l'état modifié, par exemple sous forme retardée ou sous forme pulsatile.

11. Composé pour l'utilisation selon l'une quelconque des revendications 1 à 10, dans lequel l'application systémique a lieu par voie orale, par voie perorale, par voie intramusculaire, par voie intraveineuse, par voie intrapéritonéales, par voie buccale, par voie nasale, par voie transdermique, par inhalation ou par voie rectale.

12. Composé pour l'utilisation selon l'une quelconque des revendications 1 à 11, dans lequel l'application a lieu par voie orale et le médicament est présent sous la forme de comprimés, de capsules, de dragées, de granulés, d'une solution non stérilisée ou d'une suspension ou bien l'application a lieu par voie parentérale et le médicament est présent sous la forme d'une suspension ou d'une solution stérilisée ou bien l'application a lieu par voie rectale et le médicament est présent sous la forme de suppositoires ou bien l'application a lieu par voie orale ou par voie nasale et le médicament est présent sous la forme d'un aérosol.

13. Composé pour l'utilisation selon l'une quelconque des revendications 1 à 12, dans lequel l'application a lieu par voie locale et la concentration de la substance active ou des substances actives pour l'application locale a lieu sous la forme d'agents topiques à concurrence de moins de 0,5 g/100 g à 25 g/100 g de préparation ou plus, de préférence à concurrence de 1 g/100 g à 20 g/100 g de préparation, de manière particulièrement préférée à concurrence de 1 g/100 g à 15 g/100 g de préparation, de manière tout particulièrement préférée à concurrence de 1,5 g/100 g à 10 g/100 g de préparation, et de manière de loin préférée à concurrence de 5 g/100 g à 10 g/100 g de préparation.

14. Composé pour l'utilisation selon la revendication 13, dans lequel le tarenflurbil, respectivement son sel ou son ester pharmaceutiquement acceptable est combiné à une ou plusieurs substances actives appropriées pour le traitement local de neuropathies douloureuses, dans la concentration thérapeutique habituelle, par exemple à de la capsaïcine, à de la lidocaïne ou à de la benzocaïne.

15. Composé pour l'utilisation selon la revendication 13 ou 14, dans lequel le médicament est présent sous la forme d'une formulation appropriée pour l'application topique sur la peau, par exemple sous la forme d'une pommade, d'une crème, d'un gel, d'une solution, d'une préparation liposomiale, d'un emplâtre ou sous la forme d'un bandage enduit.
